# FASCICULE DE BREVET EUROPEEN

(11) **EP 2 129 407 B1**
(45) Date de publication et mention de la délivrance du brevet: **14.11.2012**
(21) Numéro de dépôt: 08717269.8
(22) Date de dépôt: 29.02.2008
(51) Int. Cl.: A61L 27/46, A61L 27/50, A61L 27/52, A61L 24/00, A61K 9/16, A61K 49/04, A61K 49/18

(54) **COMPOSITION GRANULAIRE DESHYDRATEE ET SES APPLICATIONS BIOMEDICALES**
DEHYDRIERTE GRANULATFÖRMIGE ZUSAMMENSETZUNG UND BIOMEDIZINISCHE ANWENDUNGEN DAFÜR
DEHYDRATED GRANULAR COMPOSITION AND BIOMEDICAL APPLICATIONS THEREOF

(30) Priorité: 01.03.2007 FR 0753590
(43) Date de publication de la demande: 09.12.2009
(73) Titulaire: Biomatlante, 44360 Vigneux de Bretagne (FR)
(72) Inventeur: GOBIN, Chantal, F-44360 Vigneux de Bretagne (FR); BOURGES, Xavier, 01140 Mogneneins (FR)
(74) Mandataire: Ahner, Francis
(86) Numéro de dépôt international: PCT/EP2008/052488
(87) Numéro de publication internationale: WO 2008/107384

(56) Documents cités:
- EP-B1- 0 692 986
- WO-A-00/07639
- WO-A2-03/024211
- FR-A1- 2 737 663
- US-B1- 6 777 001
- DATABASE WPI Week 199109 Derwent Publications Ltd., London, GB; AN 1991-061942 XP002477887 & JP 03 011006 A (SANKIN KOGYO KK) 18 janvier 1991 (1991-01-18) cité dans la demande

## Description

La présente invention concerne une nouvelle composition sous forme sèche ou quasi sèche pouvant être hydratée afin de former un biomatériau de comblement injectable et utilisable en vertébroplastie, fémoroplastie et chirurgie mini invasive.

Des substituts osseux à base de particules de phosphate de calcium et d'une colle biologique sont connus de l'état de la technique.

Ainsi, G. Daculsi et al. ont décrit dans Ann. Oto. Rhino. Laryngol. 101 :1992, l'efficacité d'une composition microporeuse biphasique de phosphate de calcium pour l'amélioration de la cavité mastoïdienne.

Les mêmes auteurs ont également rapporté l'efficacité d'une composition macroporeuse de phosphate de calcium biphasique pour la préparation chirurgicale d'os longs (Journal of Biomedical Material Research Vol 24, 379-396) et dans les arthrodèses vertébrales (Clinical Orthopadics and Related Research 1989, 248, 169-175).

D'autre part, le brevet JP 3 011 006 décrit un ciment pour tissus durs comprenant une phase minérale constituée d'au moins 60% de phosphate tricalcique alpha et d'hydroxyapatite et/ou monophosphate de calcium, et une phase liquide comprenant de la carboxyméthylcellulose. Aucun dérivé cellulosique n'est présent dans la phase minérale.

Une telle composition présente cependant l'inconvénient, en raison de la solubilité trop grande du phosphate tricalcique alpha, de ne pas être suffisamment stable pour permettre un processus de résorption/substitution du tissus dur. En outre, une telle composition est susceptible de générer des processus inflammatoires néfastes. Ce mélange constitue un ionomère de calcium impropre à l'injection après quelques minutes par durcissement du mélange dès sa constitution. Cette association présente une double instabilité, une contraction volumétrique avec relargage d'eau après plusieurs jours, et surtout une chute de la viscosité après stérilisation à l'autoclave du mélange.

Le brevet EP 0 692 986 décrit une composition injectable "prête à l'emploi" d'un biomatériau de résorption/substitution de tissus de soutien dentaires, osseux, et ostéo-articulaires composée de : 40 à 75% en poids d'une phase minérale comprenant soit un mélange de phosphate tricalcique P (A) et d'hydroxyapatite (B), dans un rapport A/B compris entre 1/4 et 7/3, soit du phosphate double de calcium et de titane (Ca(Ti)₄(PO₄)₆) et de 60 à 25% en poids d'une phase liquide comprenant une solution aqueuse d'un polymère dérivé de la cellulose. Aucun dérivé cellulosique n'est présent dans la phase minérale. Malheureusement, ces gels présentent une courte durée vie, rendant leur conservation délicate. En effet, après une période de repos de une à deux semaines, la démixtion de ces compositions a été observée.

La présente invention a pour but d'éviter de tels problèmes de stabilité en fournissant une composition stérile sous forme sèche ou quasi-sèche pouvant être hydratée extemporanément afin de former un gel injectable.

Plus précisément, la composition selon l'invention est sous forme granulaire et comprend en poids 0,1 à 5% d'au moins un polymère dérivé de la cellulose, 75 à 99,9% d'une phase minérale comprenant de l'hydroxyapatite et/ou du phosphate tricalcique β, et 0 à 10% d'eau, de préférence 0 à 7% d'eau, plus préférentiellement 0 à 5% d'eau, plus préférentiellement encore environ 5% d'eau.

La composition selon l'invention comprend donc jusqu'à environ 10% d'eau, et avantageusement environ 5% d'eau.

On entend par polymère dérivé de la cellulose, un polymère hydrophile et hydrosoluble de cellulose, dont les groupements hydroxyles ont été remplacés par divers substituants.

Le polymère dérivé de la cellulose peut être choisi dans le groupe comprenant l'hydroxypropylméthylcellulose, la méthylcellulose, l'hydroxyéthylcellulose et la carboxyméthylcellulose.

Selon un mode de réalisation particulièrement avantageux de l'invention, le polymère dérivé de la cellulose est l'hydroxypropylméthylcellulose (HPMC).

Avantageusement, le ratio en poids hydroxyapatite/phosphate tricalcique β dans la phase minérale est compris entre 10/90 et 90/10, plus avantageusement encore compris entre 20/80 et 80/20.

Selon un mode de réalisation particulièrement avantageux de l'invention, la phase minérale selon l'invention comprend en poids 60% d'hydroxyapatite et 40% de phosphate tricalcique β.

Avantageusement encore, l'hydroxyapatite et le phosphate tricalcique β présentent une granulométrie comprise entre 40 et 200 µm.

Un avantage supplémentaire de l'invention tient au fait que la composition peut être hydratée extemporanément avec diverses solutions aqueuses.

L'invention a donc également pour objet l'utilisation d'une composition granulaire telle que décrite précédemment pour la fabrication extemporanée d'un hydrogel caractérisé en ce qu'elle consiste à mélanger la composition granulaire selon l'invention à un liquide choisi dans le groupe comprenant l'eau, le sérum physiologique, le plasma ou fluides biologiques contenant ou pas des produits issus de l'ingénierie tissulaire, PRP (Platelet Rich Plasma), moelle osseuse, ou encore les solutions d'agent de contraste pour l'imagerie par résonance magnétique nucléaire (IRM) ou les solutions d'agent radio opaque pour l'imagerie par rayons X.

Les compositions selon l'invention présentent une grande utilité pour de nouvelles techniques chirurgicales de plus en plus pratiquées, comme la vertébroplastie ou la kyphoplastie. Ces opérations nécessitent des biomatériaux de nature radio opaque (de façon permanente ou non suivant les cas). Elles sont en effet réalisées sur des vertèbres plus ou moins fracturées et la fuite de matériau en dehors des vertèbres peut être critique, donnant lieu à des complications neurologiques et vasculaires pouvant entraîner la mort. Il est donc indispensable que le chirurgien puisse observer la quantité de produit injecté, sa dispersion dans la vertèbre et surtout vérifier que le matériau reste bien à l'intérieur de celle-ci.

Le volume de liquide ne doit pas être supérieur à ce que la composition granulaire puisse absorber afin d'éviter la démixtion *in vivo* de la poudre.

La quantité de liquide ajoutée à la composition granulaire est ajustée afin d'obtenir un gel injectable.

En raison de la grande variété de solutions pouvant être ajoutées à la composition granulaire, la quantité de liquide est exprimée en un volume (ml) ajouté à un poids (g) de composition granulaire.

Préférentiellement, la proportion de liquide par rapport à la proportion de composition granulaire est comprises entre 35 ml de liquide pour 65 g de composition granulaire et 55 ml de liquide pour 45 g de composition granulaire, plus préférentiellement entre 40 ml de liquide pour 60 g de composition granulaire et 50 ml de liquide pour 50 g de composition granulaire, plus préférentiellement encore égales à 45 ml de liquide pour 55 g de composition granulaire.

Selon l'invention, les agents radio opaques pour l'imagerie médicale par rayons X peuvent être de façon avantageuse des composés iodés, ioniques ou non ioniques tels que le loméron® ou le Iopamidol®. Ces solutions iodées rendent le gel totalement radio opaque et permettent donc de le détecter par radiographie aux rayons X.

Les agents radio opaques présents dans le gel injecté présentent l'avantage supplémentaire de disparaître au cours du temps, ce qui permet d'évaluer l'efficacité de la reconstruction osseuse.

De plus, les substituts osseux, obtenus après l'hydratation des compositions selon l'invention par une solution iodée, présentent des propriétés rhéologiques exceptionnelles. Ils forment des produits visqueux, de structure épaisse sous forme de pâte injectable (type pâte de dentifrice).

Les agents de contraste pour l'imagerie médicale par résonance magnétique nucléaire peuvent être utilisés, notamment en cas d'allergie du patient à l'iode. Avantageusement, de tels agents de contraste contiennent du gadolinium(III), comme par exemple le produit ProHance®.

Les hydrogels contenant un agent de contraste pour l'IRM peuvent être observés après l'injection. Le chirurgien peut ainsi vérifier leur bonne implantation. Les agents de contraste sont ensuite rapidement éliminés du corps du patient.

L'invention a également pour objet les nouveaux gels obtenus par hydratation des compositions sèches, notamment les gels contenant les agents de contraste.

L'invention a également pour objet l'utilisation de ces hydrogels en tant que substitut osseux.

L'invention a aussi pour objet le procédé de préparation des compositions précédemment décrites. Un tel procédé comprend les étapes successives suivantes :
- préparation d'un mélange homogène comprenant la phase minérale et la solution aqueuse de polymère dérivé de la cellulose ;
- déshydratation du mélange, par exemple à l'aide d'un lyophiliseur ou d'une étuve ;
- éventuellement désagglomération du produit déshydraté, par exemple sur un tamis de 300 µm ;
- stérilisation du produit déshydraté, par exemple à l'autoclave.

Avantageusement, la température de stérilisation est de 121°C.

L'invention va maintenant être illustrée de façon non limitative par les exemples suivants.

### Exemple 1 de composition :

Un substitut osseux a été préparé ainsi :
30 g d'hydroxypropylméthylcellulose (HPMC) de type E4M ont été dissous dans 970 g de solution physiologique à l'aide d'un agitateur mécanique pendant 24 heures.

Des granules de céramique de phosphate de calcium MBCP® (Biomatlante), contenant en poids 60% d'hydroxyapatite et 40% de phosphate tricalcique β, de taille comprise entre 80 et 200 µm ont été préparés par tamisage humide.

Les granules ont été ajoutés à la solution d'HPMC dans les proportions suivantes en poids : 45% de la solution d'HPMC et 55% de granules. Le mélange a été effectué à l'aide d'un malaxeur mécanique.

Le substitut osseux a ensuite été lyophilisé jusqu'à un taux d'humidité inférieur à 5%, et 5,5 g du produit lyophilisé ont été disposés dans des seringues en polycarbonate de 12 ml disposant d'un système laissant passer la vapeur d'eau. Les seringues contenant le substitut osseux ont été stérilisées à l'autoclave à 121°C pendant 30 minutes.

Après la stérilisation, le substitut osseux a été reconstitué par 4,5 ml de solution radio opaque Iomeron® 250. Le produit radio opaque a été prélevé à l'aide d'une seringue de 12 ml et connectée à la seringue contenant le substitut osseux lyophilisé à l'aide d'un connecteur « luer lock » femelle-femelle. Le produit de contraste a été injecté à l'intérieur de la seringue contenant le substitut osseux, et l'ensemble a été passé plusieurs fois d'une seringue à l'autre afin d'homogénéiser le produit final. Le substitut osseux contenant le produit de contraste est alors prêt à être utilisé.

Il a été vérifié sous scopie que la radio-opacité du produit ainsi préparé était suffisante et que le fait de rajouter le produit de contraste ne changeait pas la biocompatibilité, ni n'influençait la repousse osseuse.

### Vérification in vivo de l'innocuité du gel hydraté chez le lapin New Zealand :

Un substitut osseux a été préparé en hydratant avec de l'eau stérile, une composition sèche contenant en poids 94,2% de granules de phosphate de calcium biphasé constitué de 60% d'hydroxyapatite et 40% de phosphate tricalcique β de taille comprise entre 80 et 200 µm, 2,3% d'hydroxypropylméthylcellulose et 3,5% d'eau.

Un substitut osseux contenant du Ioméron® a également été préparé comme décrit précédemment.

### Protocole opératoire

Les animaux ayant participé à l'étude étaient tous matures. Le site d'injection retenu était le site endo médullaire fémoral.

Chaque animal a été opéré des deux fémurs. Le membre opéré a été tondu quelques minutes avant l'intervention.

Le protocole d'anesthésique a été le suivant :
- 15 minutes avant l'intervention, injection intra musculaire de 250 mg de chlorhydrate de kétamine.
- 1 à 2 minutes avant l'intervention, anesthésie locale et intramusculaire du genou, à l'aide d'un mélange de chlorhydrate de lidocaine à 1% (Xylocaine adrénaline, Astra France, Nanterre) dans des proportions respectives de 2/3 - 1/3.

L'intervention a été effectuée dans des conditions d'asepsie stricte.

L'animal anesthésie est placé en décubitus dorsal, les quatre membres attachés. Un billot est glissé sous le genou du côté opéré pour maintenir une flexion d'environ 30° de ce genou. Le site opératoire est ensuite isolé par un champ stérile percé.

Les étapes sont les suivantes :
- Incision cutanée para-patellaire interne d'environ 3 cm.
- Incision de l'aileron patellaire interne et de la capsule articulaire.
- Forage, à l'aide d'une mèche de 6 mm de diamètre, d'un canal dans la gorge trochléenne.
- Hémostase à l'aide d'une compresse stérile.
- Injection lente des substituts osseux préparés (avec ou sans Iomeron®) comme décrit précédemment.
- Il est disposé ensuite un bouchon de MBCP de diamètre 6,5 mm afin d'éviter que les substituts osseux ne s'écoulent du site d'implantation.
- Fermeture, en un seul plan, de la capsule et de l'aileron patellaire par un surget étanche de vicryl.
- Fermeture cutanée par un surget étanche de vicryl.
- Mise en place d'un gel désinfectant de bétadine sur la suture.
- Mise en place d'une bande d'élastoplaste.

Aucune antibiothérapie n'a été utilisée en per opératoire ni en post opératoire.

Tous les animaux ont été sacrifiés 6 semaines après implantation par une injection létale intraveineuse de 0,25 gramme de thiopental sodique (Nesdonal, Spécia Rhone Poulenc Paris)

Les implants ont été récupérés et ont été traités afin de les inclure dans une résine de GMA. Différentes analyses ont été menées.

### Résultats d'analyses histologiques

Des analyses quantitatives ont permis de déterminer le taux de repousse osseuse, la résorption du matériau et la qualité de l'os néoformé. Les techniques utilisées ont été la microtomodensitométrie, la microscopie polarisée, et la microscopie à balayage électronique.

Aucune différence dans la repousse osseuse entre le MBCP Gel sec reconstitué avec de l'eau stérile ou avec du Iomeron® n'est observable.

L'innocuité des 2 produits a été confirmée et aucune réaction de rejet d'un corps étranger n'a été observée.

### Exemple 2 de reconstitution

Un substitut osseux injectable MBCP/HPMC lyophilisé identique à l'exemple 1 précédent a été préparé dans des seringues de 1 ml (0,55g de gel lyophilisé).

Après stérilisation le substitut osseux a été reconstitué par 4 types de solutions d'origine biologique :

| | | |
|---|---|---|
| i) | Plasma de culture cellulaire, sérum Bovin | 0,45 ml |
| ii) | Sérum humain obtenu par centrifugation et concentré plaquettaire (PRP) | 0,45 ml |
| iii) | Sang (lapin) séparé sur Ficoll (sans lignée rouge) | 0,50 ml |
| iv) | Moelle osseuse totale (lapin) | 0,50 ml |

Les 4 solutions ont été préparées dans des seringues de 1 ml avec Luer lock. La seringue contenant le MBCP gel lyophilisé a été connectée à la seringue contenant le fluide biologique ou les dérivés cellulaires, et son contenu injecté dans le substitut osseux. L'ensemble a été passé plusieurs fois d'une seringue à l'autre afin d'homogénéiser le produit final.

L'injectabilité/extrusion a été observée. Il a été noté que la reconstitution du MBCP gel lyophilisé avec des fluides biologiques, contenant ou non des éléments figurés tel que plaquettes, ou cellules est possible et confèrent au substitut osseux des propriétés suffisantes pour l'extrusion et le comblement de défauts osseux ou ostéoarticulaires.

## Revendications

1. Composition granulaire pour biomatériau, **caractérisée en ce qu'**elle comprend en poids 0,1 à 5% d'au moins un polymère dérivé de la cellulose, 75 à 99,9% d'une phase minérale comprenant de l'hydroxyapatite et/ou du phosphate tricalcique β et 0 à 10% d'eau.

2. **Composition selon la revendication 1, caractérisée en ce qu'elle comprend de** 0 à 7% d'eau.

3. **Composition selon l'une quelconque des revendications précédentes, caractérisée en ce qu'elle comprend** environ 5% d'eau.

4. Composition selon la revendication 1, **caractérisée en ce que** le polymère dérivé de la cellulose est choisi dans le groupe comprenant l'hydroxypropylméthylcellulose, la méthylcellulose, l'hydroxyéthylcellulose et, la carboxyméthylcellulose.

5. Composition selon l'une quelconque des revendications précédentes, **caractérisée en ce que** le ratio en poids hydroxyapatite/phosphate tricalcique β dans la phase minérale est compris entre 10/90 et 90/10.

6. **Composition selon la revendication 5, caractérisée en ce que le ratio en poids hydroxyapatite/phosphate tricalcique β dans la phase minérale est compris entre** 20/80 et 80/20.

7. **Composition selon la revendication 5 ou 6, caractérisée en ce que le ratio en poids hydroxyapatite/phosphate tricalcique β dans la phase minérale est compris d'** environ 60/40.

8. Composition selon l'une quelconque des revendications précédentes, **caractérisée en ce que** l'hydroxyapatite et le phosphate tricalcique β présentent une granulométrie comprise entre 40 et 200 µm.

9. Utilisation d'une composition granulaire selon l'une quelconque des revendications précédentes pour la fabrication extemporanée d'un hydrogel, **caractérisée en ce qu'**elle consiste à mélanger la composition granulaire à un liquide choisi dans le groupe comprenant l'eau, le sérum physiologique, le plasma sanguin ou les PRP, la moelle osseuse, les dérivés de l'ingénierie tissulaire, les solutions d'agent de contraste pour l'imagerie par résonance magnétique nucléaire et les solutions de composé radio opaque pour l'imagerie par rayons X.

10. Utilisation selon la revendication 9, **caractérisée en ce que** la proportion de liquide par rapport à la proportion de composition granulaire est comprises entre 35 ml de liquide pour 65 g de composition granulaire et 55 ml de liquide pour 45 g de composition granulaire.

11. **Utilisation selon la revendication 9, caractérisée en ce que la proportion de liquide par rapport à la proportion de composition granulaire est comprise** entre 40 ml de liquide pour 60 g de composition granulaire et 50 ml de liquide pour 50 g de composition granulaire.

12. Utilisation selon l'une quelconque des revendications 9 à 11, **caractérisée en ce que** les agents de contraste pour l'imagerie médicale par rayons X sont des composés iodés, ioniques ou non ioniques.

13. Utilisation selon l'une quelconque des revendications 9 à 12, **caractérisée en ce que** les agents de contraste pour l'imagerie médicale par résonance magnétique nucléaire contiennent du gadolinium(III).

14. Hydrogel obtenu par l'utilisation selon l'une quelconque des revendications 9 à 138.

15. Procédé de fabrication d'une composition selon l'une quelconque des revendications 1 à 8, **caractérisé en ce qu'**il comprend les étapes successives suivantes :
- préparation d'un mélange homogène aqueux comprenant la phase minérale et au moins un polymère dérivé de la cellulose ;
- déshydratation du mélange, par exemple à l'aide d'un lyophiliseur ou d'une étuve ;
- éventuellement désagglomération du produit déshydraté ;
- stérilisation du produit déshydraté.

## Claims

1. Granular composition for a biomaterial, **characterized in that** it comprises by weight 0.1 to 5% of at least one polymer derived from cellulose, 75 to 99.9% of a mineral phase comprising hydroxyapatite and/or β tricalcium phosphate and 0 to 10% of water.

2. Composition according to claim 1, **characterized in that** it includes 0 to 7% water.

3. Composition according to any of the preceding claims, **characterized in** it includes around 5% water.

4. Composition according to claim 1, **characterized in that** the polymer derived from cellulose is chosen from the group including hydroxypropylmethylcellulose, methylcellulose, hydroxyethylcellulose and carboxymethylcellulose.

5. Composition according to any one of the previous claims, **characterized in that** the hydroxyapatite/β tricalcium phosphate weight ratio in the mineral phase is comprised between 10/90 and 90/10.

6. Composition according to claim 5, **characterized in that** the hydroxyapatite/β tricalcium phosphate weight ratio in the mineral phase is comprised between 20/80 and 80/20.

7. Composition according to claim 5 or 6, **characterized in that** the hydroxyapatite/β tricalcium phosphate weight ratio in the mineral phase is comprised about 60/40.

8. Composition according to any of the preceding claims, **characterized in that** hydroxyapatite and β tricalcium phosphate have a grain size comprised between 40 and 200 µm.

9. Use of a granular composition according to any of the preceding claims, for making a hydrogel extemporaneously, **characterized in that** it consists of mixing the granular composition with a liquid selected from the group comprising water, saline, blood plasma or PRPs, bone marrow, derivatives from tissue engineering, contrast agent solutions for nuclear magnetic resonance imaging and radio-opaque compound solutions for X-ray imaging.

10. Use according to claim 9, **characterized in that** the liquid proportion relative to the granular composition proportion, is comprised between 35 mL of liquid for 65 g of granular composition and 55 mL of liquid for 45 g of granular composition.

11. Use according to claim 9, **characterized in that** the liquid proportion relative to the granular composition proportion, is comprised between 40 mL of liquid for 60 g of granular composition and 50 mL of liquid for 50 g of granular composition.

12. Use according to any one of claims 9 to 11, **characterized in that** the contrast agents for medical X-ray imaging are ionic or non-ionic iodinated compounds.

13. Use according to any one of claims 9 to 12, **characterized in that** the contrast agents for medical nuclear magnetic resonance imaging contain gadolinium(III).

14. Hydrogel obtained by the use according to any of claims 9 to 13.

15. A process for making a composition according to any of claims 1 to 8, **characterized in that** it comprises the following successive steps:
- preparation of an aqueous homogenous mixture comprising the mineral phase and at least one polymer derived from cellulose;
- dehydration of the mixture, for example with a freeze drier or an oven;
- optional deagglomeration of the dehydrated product;
- sterilization of the dehydrated product.

## Patentansprüche

1. Granulatförmige Zusammensetzung für Biomaterial, **dadurch gekennzeichnet, dass** sie, bezogen auf das Gewicht, 0,1 bis 5% mindestens eines von Cellulose abgeleiteten Polymers, 75 bis 99,9% einer Mineralphase, die Hydroxyapatit und/oder Tricalciumphosphat β aufweist, und 0 bis 10% Wasser aufweist.

2. Zusammensetzung nach Anspruch 1, **dadurch gekennzeichnet, dass** sie 0 bis 7% Wasser aufweist.

3. Zusammensetzung nach einem der vorstehenden Ansprüche, **dadurch gekennzeichnet, dass** sie ungefähr 5% Wasser aufweist.

4. Zusammensetzung nach Anspruch 1, **dadurch gekennzeichnet, dass** das von Cellulose abgeleitete Polymer ausgewählt ist aus der Gruppe umfassend Hydroxypropylmethylcellulose, Methylcellulose, Hydroxyethylcellulose und Carboxymethylcellulose.

5. Zusammensetzung nach einem der vorstehenden Ansprüche, **dadurch gekennzeichnet, dass** in der Mineralphase das Gewichtsverhältnis Hydroxyapatit/Tricalciumphosphat β zwischen 10/90 und 90/10 liegt.

6. Zusammensetzung nach Anspruch 5, **dadurch gekennzeichnet, dass** in der Mineralphase das Gewichtsverhältnis Hydroxyapatit/Tricalciumphosphat β zwischen 20/80 und 80/20 liegt.

7. Zusammensetzung nach Anspruch 5 oder 6, **dadurch gekennzeichnet, dass** in der Mineralphase das Gewichtsverhältnis Hydroxyapatit/Tricalciumphosphat β ungefähr 60/40 beträgt.

8. Zusammensetzung nach einem der vorstehenden Ansprüche, **dadurch gekennzeichnet, dass** der Hydroxyapatit und das Tricalciumphosphat β eine Körnung zwischen 40 und 200µm aufweisen.

9. Verwendung einer granulatförmigen Zusammensetzung nach einem der vorstehenden Ansprüche zur extemporanen Herstellung eines Hydrogels, **dadurch gekennzeichnet, dass** sie darin besteht, die granulatförmige Zusammensetzung mit einer Flüssigkeit zu mischen, wobei die Flüssigkeit ausgewählt ist aus der Gruppe umfassend Wasser, physiologisches Serum, Blutplasma oder PRP, Knochenmark, Derivate der Gewebekonstruktion, Kontrastmittellösungen für NMR-Bildgebungsverfahren (NMR: nuklear magnetische Resonanz), Lösungen einer strahlenopaken Verbindung für Röntgenstrahlen-Bildgebungsverfahren.

10. Verwendung nach Anspruch 9, **dadurch gekennzeichnet, dass** das Verhältnis von Flüssigkeit zu granulatförmiger Zusammensetzung zwischen 35ml Flüssigkeit auf 65g granulatförmige Zusammensetzung und 55ml Flüssigkeit auf 45g granulatförmige Zusammensetzung liegt.

11. Verwendung nach Anspruch 9, **dadurch gekennzeichnet, dass** das Verhältnis von Flüssigkeit zu granulatförmiger Zusammensetzung zwischen 40ml Flüssigkeit auf 60g granulatförmige Zusammensetzung und 50ml Flüssigkeit auf 50g granulatförmige Zusammensetzung liegt.

12. Verwendung nach einem der Ansprüche 9 bis 11, **dadurch gekennzeichnet, dass** die Kontrastmittel für medizinische Röntgenstrahlen-Bildgebungsverfahren iodierte, ionische oder nichtionische Verbindungen sind.

13. Verwendung nach einem der Ansprüche 9 bis 12, **dadurch gekennzeichnet, dass** die Kontrastmittel für medizinische NMR-Bildgebungsverfahren Gadolinium(III) enthalten.

14. Durch die Verwendung nach einem der Ansprüche 9 bis 13 gewonnenes Hydrogel.

15. Verfahren zur Herstellung einer Zusammensetzung nach einem der Ansprüche 1 bis 8, **dadurch gekennzeichnet, dass** es der Reihe nach die folgenden Schritte aufweist:
- Erzeugen eines homogenen wässrigen Gemisches, das die Mineralphase und mindestens ein von Cellulose abgeleitetes Polymer aufweist;
- Dehydrieren des Gemisches, zum Beispiel mit Hilfe eines Gefriertrockners oder eines Trockenofens;
- gegebenenfalls Entklumpen des dehydrierten Produkts;
- Sterilisieren des dehydrierten Produkts.
